Europäisches Patentamt

European Patent Office

Office européen des brevets

(19)

(11) Publication number: **0 113 526
B1**

(12) **EUROPEAN PATENT SPECIFICATION**

(45) Date of publication of patent specification: **19.03.86**

(21) Application number: **83307263.0**

(22) Date of filing: **29.11.83**

(51) Int. Cl.⁴: **C 08 J 7/04,** B 29 B 13/02,
B 29 B 15/10, A 61 B 19/04 //
A61F5/42

(54) **Method of coating rubber or polymer articles.**

(30) Priority: **30.11.82 US 445760**

(43) Date of publication of application:
**18.07.84 Bulletin 84/29**

(45) Publication of the grant of the patent:
**19.03.86 Bulletin 86/12**

(84) Designated Contracting States:
**AT BE CH DE FR GB IT LI LU NL SE**

(56) References cited:
**FR-A-1 434 453
FR-A-2 293 486
GB-A-1 268 637
GB-A-1 496 345
US-A-3 813 695**

(73) Proprietor: **LRC PRODUCTS LIMITED
North Circular Road
London, E4 8QA (GB)**

(72) Inventor: **Podell, Howard Irwin
28 Beachfront Lane
New Rochelle New York 10805 (US)**
Inventor: **Goldstein, Albert
87, Glenwood Drive
Tinton Falls New Jersey 07724 (US)**

(74) Representative: **Austin, Hedley William et al
A.A. THORNTON & CO. Northumberland House
303-306 High Holborn
London WC1V 7LE (GB)**

Courier Press, Leamington Spa, England.

**Description**

The present invention is concerned with a method of coating polymer articles (particularly rubber articles) and, more specifically, with a method of producing a lubricating layer comprising a hydrogel polymer on such an article.

U.S. Patent 3813695 ("the Podell patent") describes a surgeon's glove in which the glove material is formed of a laminate consisting of an outer layer of flexible material, for example rubber, and an inner layer of hydrophilic plastic material (such as a hydrogel polymer), the inner and outer layers being bonded together.

There are many known hydrogel polymers, examples of which are polyvinyl pyrrolidone, polyhydroxyethyl acrylate or methacrylate, polyhydroxypropyl acrylate or methacrylate, and copolymers of these with each other or with acrylic or methacrylic acid, acrylic or methacrylic esters or vinyl pyridine, and polymers of acrylamides.

There are many disclosures of the coating of rubber and other polymer articles, such as catheters and bathing caps, with such hydrogel polymers by dipping in a solution of a hydrophilic, hydrogel-forming polymer and curing the resulting polymer layer.

Examples of such disclosures include U.S. Patents 3326742, 3585103, 3607473, 3745042, 3901755, 3925138, 3930076, 3940533, 3966530, 4024317, 4110495 and 4125477, and British Patents 1028446 and 859297.

Our prior copending European Patent Application No. 83305099.0 describes and claims a specific process of producing a lubricating layer comprising a hydrogel polymer on a flexible rubber article in which a solution of a hydrophilic hydrogel-forming polymer is applied to a rubber surface.

We have found that good adhesion can be obtained, and process simplification achieved, if the solution of hydrogel-forming polymer also contains a trivalent cation.

According to the invention, therefore, there is provided a method of producing a lubricating layer comprising a hydrogel polymer on a flexible rubber article, which comprises applying to the article a solution having dissolved therein a hydrophilic hydrogel-forming polymer and a salt of a trivalent cation, and curing the polymer.

The trivalent cation may be, for example, aluminium or ferric iron; aluminium is preferred.

The solution containing the polymer and the salt of the trivalent cation may be an aqueous solution or a solution in an organic solvent (such as an alcohol). The solution is preferably applied to the rubber article by dipping, especially where the rubber article is itself formed by dipping, as this enables the process to be carried out in continuous operation.

The solution preferably contains a curing agent for the polymer, and optionally an acid catalyst, such as para-toluene sulphonic acid or phosphoric acid, or an ammonium salt which thermally decomposes to form an acid (e.g. ammonium phosphate).

The rubber article may, in some embodiments, be treated with dilute acid before application of the solution containing the hydrogel-forming polymer; alternatively or additionally, such an acid may be present in the solution containing the polymer and the salt of a trivalent cation.

The method according to the invention is typically carried out as follows:

(a) forming a rubber article by dipping a former in a rubber latex,

(b) leaching the rubber article in hot water,

(c) optionally, priming the rubber surface of the article on the former, for example, by means of a dilute acid, and rinsing the primed surface in water or aqueous alkali,

(d) dipping said article, while still on the former, in a solution containing a salt of a trivalent cation and a hydrophilic, hydrogel-forming polymer, together with a curing agent therefor,

(e) heat drying the resulting coating such that the resulting hydrogel polymer adheres to the rubber surface,

(f) vulcanising the rubber and simultaneously curing the polymer by application of heat,

(g) stripping the resulting article from the former,

(h) applying a solution of surfactant material containing silicone to the article (for example, by tumbling in such a solution), optionally after washing, and

(i) heating the resulting coating of surfactant material so as to fix the slip properties of the coating.

The application of a solution of surfactant material provides substantially tack-free outer and inner surfaces and enhanced slip properties with respect to both damp and dry skin.

It is accordingly preferred that the lubricating layer should have surfactant material applied thereto; preferred surfactants and methods of application are described in detail in the above-mentioned copending application.

The hydrogel-forming polymer used in hhe method according to the invention may be, for example, a copolymer of 2-hydroxyethyl methacrylate (HEMA). (The term "copolymer" includes binary copolymers and also ternary and higher copolymers.) The HEMA copolymer may be, for example, a binary copolymer of HEMA with acrylic acid, methacrylic acid (MAA) or 2-ethylhexyl acrylate (EHA), or a ternary copolymer of HEMA, MAA and EHA. Particularly preferred classes of HEMA copolymers are described in detail in the above-mentioned copending application.

Other hydrogel-forming polymers which may be used are copolymers of other hydroxyalkyl acrylates or methacrylates, in which the hydroxyalkyl group may be, for example, hydroxypropyl or 2,3 - di - hydroxypropyl; copolymers of N-vinyl 2-pyrrolidone, glyceryl acrylate or methacrylate; graft copolymers of polyvinyl alcohol, or partially hydrolysed polyacrylonitrile, polyacrylamide or polymethacrylamide.

The rubber on which the lubricating layer is produced according to the present invention may be a natural or synthetic rubber; natural rubber is preferred. It is also preferred that the rubber article should be formed (prior to bonding of the lubricating layer formed from hydrogel polymer thereto) by dipping of a rubber latex. The hydrogel layer is preferably applied to the rubber before vulcanisation thereof; this has the surprising result that the skin-contacting surface has a much cooler feel. This may be because there is greater vapour transmission through the final coated article.

The method according to the present invention is particularly useful for producing a lubricating (skin-contacting) layer on surgeons gloves. However, the method is also applicable to other skin- or tissue-contacting flexible rubber articles, such as condoms, gloves used by doctors and veterinary surgeons for examination purposes, catheters, urethers, sheets and sheath-type incontinence devices.

When the method according to the present invention is used for articles such as urethers and catheters, the lubricating layer formed from hydrogel polymer is provided on the outer surface (this being the skin-contacting surface); for condoms the layer formed from hydrogel polymer may be provided on the inner surface and/or on the outer surface.

According to a modification of the method of the invention, the lubricating layer can be applied to a (non-elastomeric) polymer article rather than a rubber article. Examples of such polymer articles include fibres and fabrics of polyester, nylon or acrylic polymers, whereby improved adhesion of hydrogel polymer layers can be achieved.

In order that the present invention may be more fully understood, the following Example is given by way of illustration only.

Example

A series of hand-shaped formers were dipped into a coagulant (a calcium salt solution), dried and then dipped into a natural rubber latex to produce a thin rubber layer on each former. The coated formers were then dried by heating in air for a few minutes and dipped for several minutes in water at about 70°C.

The coated formers were then rinsed in running water and dipped in a solution having the following composition:

| | |
|---|---|
| alum hydrate (including 14 parts of water per molecule of aluminium sulphate) | 28.4 grams |
| polymer A | 227 grams |
| cross-linking agent A | 80 grams |
| 85% phosphoric acid | 2.14 grams |
| water to make | 3400 grams |
| ammonium hydroxide | to adjust pH to about 5.5. |

Polymer A was a copolymer of 90% 2 - hydroxyethyl methacrylate and 10% acrylic acid; cross-linking agent A was partially methylated melamine-formaldehyde resin available commercially as Cymel 373. The solution described was prepared by dissolving aluminium sulphate in water and adjusting the pH to 5.5, and then gradually mixing (with vigorous stirring) with a solution containing the polymer in the remainder of the water.

The formers were then heated in a circulating hot air oven for approximately 30 minutes (the oven temperature was initially 80 to 90°C, but rose to 150°C for at least the last 10 minutes); this resulted in cure of the polymer and vulcanisation of the rubber.

The resulting coated gloves on formers were dipped into a bath of water containing a little soap, and the gloves were stripped from the formers under running water. The stripped gloves were rinsed in a surfactant solution containing 1% of a silicone emulsion available commercially as SAG-10 (0.1% solids). The gloves were finally air-dried in a hot oven for 30 minutes.

The dried gloves had excellent slip when donned on relatively dry hands and required no additional lubricant or powder for lubrication of the skin-contacting surface of the glove. The outer surface of the gloves were tack-free (the finger sections could be squeezed together manually, without sticking to one another).

The moisture transmission properties at 25°C (100% RH) of such a glove and an otherwise similar, but uncoated (control) glove are set out below.

| Sample | Rate of moisture transmission $(g/m^2/mm/24\ hrs)$ |
|---|---|
| According to the invention | 9.76 |
| Control | 4.22 |

The moisture transmission property (tested in the same way) for a sample produced using a concentrated ethanolic solution of aluminium nitrate and polymer instead of an aqueous solution containing aluminium sulphate and polymer was 7.73 $g/m^3/mm/24$ hrs.

Similar results were obtained when aluminium chloride or nitrate were used instead of aluminium sulphate, and when the polymer (and the aluminium salt) were applied in ethanolic solution instead of an aqueous solution.

Similar results were also obtained with the use of ferric sulphate instead of aluminium sulphate; a only problem was that the ferric salt tended to stain the glove.

When salts of divalent cations, such as calcium, zinc or ferrous salts, were used instead of aluminium salts, the resulting gloves exhibited little slip and could not be satisfactorily donned on dry hands.

Similar results were obtained using other hydrogel-forming polymers instead of polymer A; particularly preferred such copolymers are copolymers of 2-hydroxyethyl methacrylate with methacrylic acid and/or 2-ethylhexyl acrylate, for example, as described in the abovementioned copending application.

## Claims

1. A method of producing a lubricating layer comprising a hydrogel polymer on a flexible rubber article, characterized by applying to the article a solution having dissolved therein a hydrophilic hydrogel-forming polymer and a salt of a trivalent cation, and curing the polymer.

2. A method according to claim 1, in which the trivalent cation is aluminium.

3. A method according to claim 1 or 2, in which the solution is applied by dipping.

4. A method according to any of claims 1 to 3, in which the rubber article is treated with dilute acid prior to application of said solution.

5. A method according to any of claims 1 to 3, in which said solution further contains dilute acid.

6. A method according to any of claims 1 to 5, in which the flexible rubber article comprises dipped rubber present on a former and the rubber is vulcanised after application of said solution.

7. A method according to claim 6, in which the rubber vulcanisation and polymer curing are carried out simultaneously.

8. A method according to any of claims 1 to 7, in which the lubricating layer has surfactant material applied thereto.

9. A method according to any of claims 1 to 8, in which the polymer is a copolymer of 2-hydroxyethyl methacrylate.

10. A flexible rubber article having thereon a lubricating layer produced according to any of claims 1 to 9, which article is in the form of a glove and said lubricating layer provides an inner skin-contacting surface.

11. A modification of the method claimed in any of claims 1 to 5, in which instead of a flexible rubber article there is used a synthetic polymer article.

## Patentansprüche

1. Verfahren zur Herstellung einer Schmierschicht, welche ein Hydrogel-polymer umfaßt, auf einem flexiblen Gummigegenstand, gekennzeichnet durch Aufbringen einer Lösung, welche darin ein hydrophiles Hydrogel bildendes Polymer und ein Salz eines dreiwertigen Kations gelöst enthält, auf den Gegenstand und Aushärten des Polymers.

2. Verfahren nach Anspruch 1, worin das dreiwertige Kation Aluminium ist.

3. Verfahren nach Anspruch 1 oder 2, worin die Lösung durch Eintauchen aufgebracht wird.

4. Verfahren nach einem der Ansprüche 1 bis 3, worin der Gummigegenstand mit verdünnter Säure vor dem Aufbringen der Lösung behandelt wird.

5. Verfahren nach einem der Ansprüche 1 bis 3, worin die Lösung weiterhin verdünnte Säure enthält.

6. Verfahren nach einem der Ansprüche 1 bis 5, worin der flexible Gummigegenstand getauchten Gummi, der auf einem Former vorliegt, umfaßt und der Gummi nach dem Aufbringen der Lösung vulkanisiert wird.

7. Verfahren nach Anspruch 6, worin die Gummivulkanisation und das Polymeraushärten gleichzeitig durchgeführt werden.

8. Verfahren nach einem der Ansprüche 1 bis 7, worin auf die Schmierschicht ein oberflächenaktives Material aufgebracht wird.

9. Verfahren nach einem der Ansprüche 1 bis 8, worin das Polymer ein Copolymer von 2-Hydroxyethylmethacrylat ist.

10. Flexibler Gummigegenstand mit einer Schmierschicht darauf, hergestellt gemäß einem der Ansprüche 1 bis 9, wobei der Gegenstand in Form eines Handschuhs vorliegt und die Schmierschicht eine innere hautberührende Oberfläche zur Verfügung stellt.

11. Modifizierung des Verfahrens nach einem der Ansprüche 1 bis 5, worin anstelle eines flexiblen Gummigegenstandes ein synthetischer Polymergegenstand verwendet wird.

## Revendications

1. Procédé pour produire une couche lubrifiante comprenant un polymère pour hydrogel sur un article flexible en caoutchouc, caractérisé en ce que l'on applique sur l'article une solution contenant un polymère hydrophile formateur d'hydrogel et un sel d'un cation trivalent dissous dans la solution et on durcit le polymère.

2. Procédé selon la revendication 1, dans lequel le cation trivalent est l'aluminium.

3. Procédé selon la revendication 1 ou 2, dans lequel la solution est appliquée par trempage.

4. Procédé selon l'une quelconque des revendications 1 à 3, dans lequel l'article de caoutchouc est traité par un acide dilué avant l'application de ladite solution.

5. Procédé selon l'une quelconque des revendications 1 à 3, dans lequel ladite solution contient en outre un acide dilué.

6. Procédé selon l'une quelconque des revendications 1 à 5, dans lequel l'article flexible en caoutchouc contient du caoutchouc immergé sur un gabarit et le caoutchouc est vulcanisé après application de ladite solution.

7. Procédé selon la revendication 6, dans lequel la vulcanisation du caoutchouc et le durcissement du polymère sont effectués simultanément.

8. Procédé selon l'une quelconque des revendications 1 à 7, dans lequel la couche lubrifiante comporte une matière tensio-active qui lui est appliquée.

9. Procédé selon l'une quelconque des revendications 1 à 8, dans lequel le polymère est un copolymère de méthacrylate de 2-hydroxy-éthyle.

10. Article flexible en caoutchouc portant une couche lubrifiante produite selon l'une quelconque des revendications 1 à 9, ledit article étant sous forme d'un gant et ladite couche lubrifiante donnant une surface intérieure de contact avec la peau.

11. Modification du procédé selon l'une quelconque des revendications 1 à 5, dans laquelle on utilise un article en polymère synthétique au lieu d'un article flexible en caoutchouc.